(19) Europäisches Patentamt
European Patent Office
Office européen des brevets

(11) EP 4 228 593 B1

(12) **EUROPEAN PATENT SPECIFICATION**

(45) Date of publication and mention of the grant of the patent:
**06.11.2024 Bulletin 2024/45**

(21) Application number: **21786989.0**

(22) Date of filing: **13.10.2021**

(51) International Patent Classification (IPC):
***A61K 8/49*** *(2006.01)* ***A61P 17/10*** *(2006.01)*
***A61Q 19/00*** *(2006.01)*

(52) Cooperative Patent Classification (CPC):
**A61K 8/4933; A61P 17/10; A61Q 19/00**

(86) International application number:
**PCT/EP2021/078360**

(87) International publication number:
**WO 2022/079122 (21.04.2022 Gazette 2022/16)**

(54) **USE OF THIOPYRIDINONE COMPOUNDS FOR PREVENTING THE FORMATION OF CUTANEOUS BLACKHEADS**

VERWENDUNG VON THIOPYRIDINONVERBINDUNGEN ZUR VERHINDERUNG DER BILDUNG VON HAUTSCHWÄCHEN

UTILISATION DE COMPOSÉS DE THIOPYRIDINONE POUR PRÉVENIR LA FORMATION DE COMÉDONS CUTANÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.10.2020 FR 2010563**

(43) Date of publication of application:
**23.08.2023 Bulletin 2023/34**

(73) Proprietor: **L'OREAL**
**75008 Paris (FR)**

(72) Inventors:
- **PREVOT-GUEGUINIAT, Amélie**
  **93601 AULNAY-SOUS-BOIS (FR)**
- **MARAT, Xavier**
  **93601 AULNAY-SOUS-BOIS (FR)**
- **BILLONI, Nelly**
  **93601 AULNAY-SOUS-BOIS (FR)**

(74) Representative: **Cabinet Nony**
**11 rue Saint-Georges**
**75009 Paris (FR)**

(56) References cited:
**FR-A1- 3 045 604** **FR-B1- 2 968 661**
**FR-B1- 3 044 905**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 228 593 B1

## Description

### Technical field

The present invention relates to the field of cosmetic products, which are more particularly intended for the prevention of cutaneous blackheads, in particular for inhibiting the appearance of the black or brown colour of comedones.

More particularly, the present invention is targeted at providing the non-therapeutic cosmetic use of a specific active principle as inhibitor of the black or brown colouration of comedones. It also relates to a non-therapeutic cosmetic method for preventing the black or brown colouration of comedones comprising the topical application, to the skin, of a composition comprising said active principle.

Within the meaning of the present invention, the term "skin" is understood to denote all of the skin of the body, excluding the scalp and the mucous membranes. Preferably, the skin is targeted at the skin of the face and/or of the hands, in particular the skin of the face, more particularly the skin of the forehead and/or of the alae of the nose and/or of the chin.

### Prior art

There exist cosmetic compositions for combatting the appearance of cutaneous blackheads or for concealing them, in particular on the face. This is because this type of cutaneous imperfection, which relates to all skin typologies, is indeed generally regarded as unattractive and thus undesirable. This is because, in view of its black, indeed even brown, colour, it is highly visible and all the more so on a lightly pigmented skin.

To date, the source of the colour of the cutaneous blackhead has not been fully established. It is, on the other hand, known that the cutaneous blackhead results from the process of comedogenesis which affects pilosebaceous units of sebaceous follicle type, which are distributed predominantly on the face.

From a clinical perspective, a cutaneous blackhead is thus an open comedo (non-inflammatory lesion) characterized by distension of the hair canal, sebum retention and excessive cornification of the pilosebaceous canal. Hypercornification would result in sebum retention and in the accumulation of cell debris.

A certain number of solutions for treating cutaneous blackheads have already been provided. Thus, active principles exhibiting a cosmetic action for reducing the development of the colour of at least one blackhead have already been developed, for example certain specific antioxidants described in Patent FR 3 044 905 B1.

Generally, these compounds unfortunately require a specific formulation which can prove to be complex. In addition, some of them are capable of damaging the organoleptic properties of the composition comprising them, in particular of resulting in the development of a specific odour or of modifying its colour, over time.

Furthermore, the majority of the solutions provided to date act in particular:

- by exfoliation of the keratinized surface parts of the cutaneous blackhead,
- by dissolution of the liquids of the sebum which are blocking the comedo,
- by mechanical extraction, or
- by the use of energy (LED).

As regards the first two solutions, they do not directly attack the coloured part of the cutaneous blackhead.

As regards the extraction method, this relies on the use, for example, of a comedo suction device, a comedo extractor, or self-adhesive patches to be removed after a few minutes of contact with the skin. It exhibits the following two major disadvantages which hinder its use:

- the repeated use of this treatment method ends up creating holes at the place of the cutaneous blackheads, that is to say that the cleansed comedo becomes even more visible than the cutaneous blackhead; and
- extraction is painful and leaves red marks, even when the operation is carried out by a professional.

Consequently, the methods currently provided are targeted essentially at treating pre-existing blackheads and not at preventing the appearance of these blackheads, by inhibiting in particular the appearance of a black or brown colour at the comedones. In other words, there remains a need to provide cosmetic active principles which make it possible to prevent the appearance of the cutaneous blackhead.

There also remains a need to make available cosmetic active principles with regard to blackheads which do not detrimentally affect the organoleptic properties of the composition comprising them, in particular the odour and/or the colour of the composition, over time.

It is an object of the present invention to meet these needs.

**Summary of the invention**

Thus, according to a first aspect, the present invention relates to the cosmetic use of at least one compound corresponding to the following formula (I) or (I'):

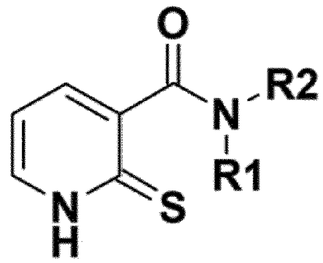

(I)

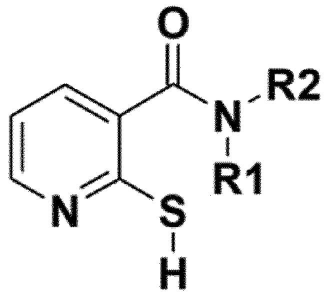

(I')

in which:

R1 and R2, which are identical or different, denote a radical chosen from:

a) a hydrogen atom;

b) a saturated linear $C_1$-$C_{20}$ or branched $C_3$-$C_{20}$ or unsaturated $C_2$-$C_{20}$ alkyl group, optionally interrupted by one or more heteroatoms chosen from N, S and O, and/or optionally substituted by one or more identical or different groups chosen from:

i) -OR3,
ii) -SR3,
iii) -NR3R4,
iv) -CONHR3, or
v) -COOR3;

c) a saturated $C_1$-$C_8$ alkyl group substituted by at least one $C_5$-$C_{12}$ aryl radical, said aryl radical optionally being substituted by one or more hydroxyls and/or by one or more $C_1$-$C_8$ alkoxy radicals; or

d) a phenyl group optionally substituted by one or more hydroxyls and/or by one or more $C_1$-$C_8$ alkoxy radicals;

R3 denoting a hydrogen atom or a saturated linear $C_1$-$C_6$ or branched $C_3$-$C_6$ or unsaturated $C_2$-$C_5$ alkyl group,
R4 denoting a hydrogen atom, a saturated linear $C_1$-$C_5$ or branched $C_3$-$C_5$ hydrocarbon group, or an acetyl group;
it being possible for R1 and R2 to form, with the nitrogen atom which bears them, a ring chosen from pyrrolidine, pyrroline, piperidine, piperazine, morpholine, thiomorpholine and azepine;
and also their salts, their solvates, their optical isomers and their racemates,
as inhibitor of the black or brown colouration of comedones.

The compounds of formula (I) or (I') which are defined above according to the invention thus make it possible to prevent the appearance of the black or brown colour of the cutaneous blackhead.

The invention thus additionally relates to the use of at least one compound of formula (I) or (I') as are defined above for preventing the formation of cutaneous blackheads.

This is because, contrary to all expectations and as emerges from the examples below, the inventors have found that the compounds of thiopyridinone type under consideration according to the invention inhibit the formation of cutaneous blackheads by intervening probably in the process for the oxidation of L-DOPA (3,4-dihydroxy-L-phenylalanine). For the record, L-DOPA is involved in the very complex melanogenesis mechanism.

More specifically, the oxidation of L-DOPA, promoted by the microbial activity present in the comedo, is expressed by a colouring effect probably of dopachrome type, the latter being a coloured intermediate of the pathway for the biosynthesis of melanin. The thiopyridinone compounds in accordance with the invention would detrimentally affect the formation of the product of the oxidation reaction in the comedones and thus would inhibit the black or brown colouration of the latter.

To the knowledge of the inventors, the compounds corresponding to the formulae (I) and (I') have already been provided in the prior art, in particular in Patent FR 2 968 661 B1 or Patent FR 3 045 604 B1, as active agents for the

skin, for the purposes of depigmenting and/or whitening the latter, and thus for an effect very distinct from that under consideration according to the invention, namely preventing the appearance of cutaneous blackheads by inhibiting the appearance of the brown or black colour in the comedones.

In addition, these distinct effects furthermore involve very different action pathways. Thus, the depigmentation of the skin is carried out by inhibition of the pathway for the biosynthesis of human melanin, in particular *via* the inhibition of the expression of tyrosinase, whereas the prevention of the brown or black colouration of the comedones is carried out, as mentioned above, by detrimentally affecting the formation of dopachrome.

Furthermore, the effectiveness of these compounds corresponding to the formulae (I) and (I') above in decolouring pigment blemishes present on the skin is also clearly not likely to be a sign of the effectiveness of these same compounds as colouring inhibitors for comedones, which is observed by the inventors.

Finally, this effectiveness of the compounds of formulae (I) and (I') in inhibiting the development of the black or brown colour of the comedones is all the more unexpected as it is specific to them. Thus, the inventors have furthermore found that certain compounds, such as, for example, kojic acid or a resorcinol derivative, 4-butylresorcinol or lucinol, known for their properties of inhibiting the pathway for the biosynthesis of human melanin, are without effect on the development of the black or brown colour of the comedones.

In the continuation of the text, the term "compound of thiopyridinone type" will be intended to denote, within the meaning of the present invention, a compound corresponding to the formula (I) or (I') which are defined above.

In particular, the compound of formula (I) or (I') as defined according to the present invention is incorporated in a cosmetic composition intended for a topical application.

Furthermore, the term "inhibitor of the black or brown colouration of the comedones" is understood to denote, within the meaning of the present invention, a compound capable of slowing down, indeed even preventing, the appearance of the black or brown colour of the comedones. In particular, a compound is regarded as inhibitor of the black or brown colouration of the comedones if the percentage of inhibition with regard to the development of the colour is at least 15%, preferably at least 20%, more particularly at least 25%, in particular as evaluated according to the protocol described in detail in the examples below. According to a second aspect, the present invention relates to a cosmetic method for preventing the black or brown colouration of the comedones, comprising the topical application, to the skin, of a cosmetic composition comprising at least one compound corresponding to the formula (I) or (I') as are defined above.

The cosmetic uses and methods defined in the present patent application are non-therapeutic. Preferably, the skin is the skin of the face and/or of the body, in particular of the face and/or of the hands, preferably of the face, and more particularly of the forehead and/or of the alae of the nose and/or of the chin.

According to a first alternative form, the skin is greasy and/or glistening skin, in particular skin exhibiting secretion and excessive excretion of sebum. According to this alternative form, the skin is in particular greasy and/or glistening skin, especially of phototype 1, 2, 3 or 4, in particular greasy and/or glistening skin of the face and/or of the hands, more particularly greasy and/or glistening skin of the forehead and/or of the alae of the nose and/or of the chin.

According to a second alternative form, the skin is non-acneic skin, namely healthy skin devoid of the clinical manifestations of acne, namely acne lesions, such as the presence of numerous acne pimples. This is because the blackheads constitute cutaneous imperfections of the skin, distinct from ailments of the skin, such as acne. In particular, the blackhead is not an inflammatory lesion of the skin, in contrast to the lesions characteristic of acne. Furthermore, the regions of the face which are favourable to the development of blackheads, namely the alae of the nose, precisely do not constitute regions partial to the development of acne.

**Brief description of the drawings**

[Fig 1] represents a graph of the measurement of the optical density at 475 nm as a function of the time for compositions 1 (containing *Cutibacterium acnes* extract at a concentration of 1.5 mg/ml), 2 (containing L-DOPA at a concentration of 125 $\mu$g/ml) and the reference composition (containing a combination of *Cutibacterium acnes* extract at a concentration of 1.5 mg/ml and of L-DOPA at a concentration of 125 $\mu$g/ml).

[Fig 2 ] represents a photograph of the surface of the epidermis magnified 7 times using a stereoscopic microscope. This image was taken after 120 hours of incubation at 32°C, in the presence of L-DOPA and of an intracellular *Cutibacterium acnes* extract (Example 1).

**Detailed description**

**COMPOUNDS OF THIOPYRIDINONE TYPE**

As mentioned above, the present invention relates to the cosmetic use of at least one compound corresponding

to the following formula (I) or (I'), referred to as "compound of thiopyridinone type":

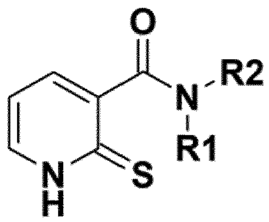

(I)

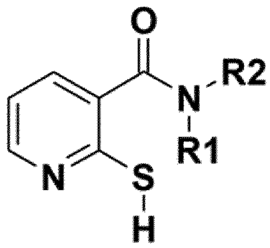

(I')

in which:

R1 and R2, which are identical or different, denote a radical chosen from:

a) a hydrogen atom;
b) a saturated linear $C_1$-$C_{20}$ or branched $C_3$-$C_{20}$ or unsaturated $C_2$-$C_{20}$ alkyl group, optionally interrupted by one or more heteroatoms chosen from N, S and O, and/or optionally substituted by one or more identical or different groups chosen from:

i) -OR3,
ii) -SR3,
iii) -NR3R4,
iv) -CONHR3, or
v) -COOR3;

c) a saturated $C_1$-$C_8$ alkyl group substituted by at least one $C_5$-$C_{12}$ aryl radical, said aryl radical optionally being substituted by one or more hydroxyls and/or by one or more $C_1$-$C_8$ alkoxy radicals; or
d) a phenyl group optionally substituted by one or more hydroxyls and/or by one or more $C_1$-$C_8$ alkoxy radicals;

R3 denoting a hydrogen atom or a saturated linear $C_1$-$C_6$ or branched $C_3$-$C_6$ or unsaturated $C_2$-$C_5$ alkyl group,
R4 denoting a hydrogen atom, a saturated linear $C_1$-$C_5$ or branched $C_3$-$C_5$ hydrocarbon group, or an acetyl group;
it being possible for R1 and R2 to form, with the nitrogen atom which bears them, a ring chosen from pyrrolidine, pyrroline, piperidine, piperazine, morpholine, thiomorpholine or azepine;
and also their salts, their solvates, their optical isomers and their racemates.

The compound (I') is the tautomeric form of the compound (I) when there exists a tautomeric equilibrium according to the following scheme:

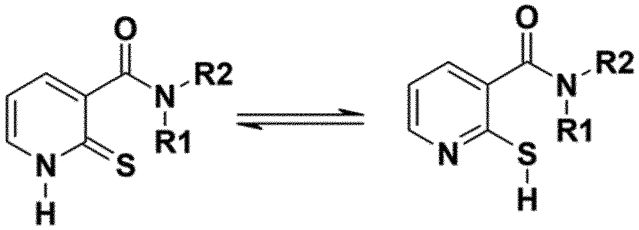

(I) (I')

The salts of the compounds of formula (I) or (I') comprise conventional non-toxic salts of said compounds, in particular those formed from an acid or from a base.
Mention may be made, as salts of the compound of formula (I) or (I') (when it comprises a quaternizable nitrogen atom), of:

a) the salts obtained by addition of the compound (I) or (I') to an inorganic acid, in particular chosen from hydrochloric acid, boric acid, hydrobromic acid, hydriodic acid, sulfuric acid, nitric acid, carbonic acid, phosphoric acid or tetrafluoroboric acid; or

b) the salts obtained by addition of the compound (I) or (I') to an organic acid, in particular chosen from acetic acid, propionic acid, succinic acid, fumaric acid, lactic acid, glycolic acid, citric acid, gluconic acid, salicylic acid, tartaric acid, terephthalic acid, methylsulfonic acid, ethylsulfonic acid, benzenesulfonic acid, toluenesulfonic acid or triflic acid.

Mention may also be made of the salts obtained by addition of the compound of formula (I) or (I') (when it comprises an acid group) to an inorganic base, in particular sodium hydroxide, potassium hydroxide, calcium hydroxide, ammonium hydroxide, magnesium hydroxide, lithium hydroxide, and sodium, potassium or calcium carbonates or hydrogencarbonates, for example, or to an organic base, in particular a primary, secondary or tertiary alkylamine, for example triethylamine or butylamine. This primary, secondary or tertiary alkylamine can comprise one or more nitrogen and/or oxygen atoms and can thus comprise, for example, one or more alcohol functional groups; mention may in particular be made of 2-amino-2-methylpropanol, ethanolamine, triethanolamine, dimethylamino-2-propanol, 2-amino-2-(hydroxymethyl)-1,3-propanediol and 3-(dimethylamino)propylamine.

Mention may also be made of amino acids, for example lysine, arginine, guanidine, glutamic acid or aspartic acid.

Advantageously, the salts of the compound of formula (I) or (I') (when it comprises an acid group) can be chosen from alkali metal or alkaline earth metal salts, in particular sodium, potassium, calcium or magnesium salts; or ammonium salts.

Advantageously, the salts of the compound of formula (I) or (I') (when it comprises a quaternizable nitrogen atom) can be chosen from halides, in particular chloride or bromide, citrate, acetate, succinate, phosphate, lactate or tartrate.

The acceptable solvates of the compounds described in the present invention comprise conventional solvates, in particular those formed during the preparation of said compounds as a result of the presence of solvents. Mention may be made, by way of example, of the solvates due to the presence of water or of linear or branched alcohols, for example ethanol or isopropanol.

The optical isomers are in particular enantiomers and diastereoisomers.

Preferably, in the context of the invention:

- "$C_t$-$C_z$", where t and z are integers, is understood to mean a hydrocarbon chain which can have from t to z carbon atoms; for example, $C_1$-$C_{20}$ denotes a hydrocarbon chain which can have from 1 to 20 carbon atoms;
- "alkyl" is understood to mean a saturated, linear or branched, aliphatic group; for example, a $C_1$-$C_{20}$ alkyl group represents a linear or branched hydrocarbon chain of 1 to 20 carbon atoms, more particularly a methyl, ethyl, propyl, isopropyl, butyl, isobutyl, tert-butyl, pentyl, hexyl, heptyl, octyl, nonyl, decyl, undecyl, dodecyl, tridecyl, tetradecyl, pentadecyl, hexadecyl, heptadecyl, octadecyl, nonadecyl or eicosyl, preferably methyl, ethyl, propyl, isopropyl, butyl, isobutyl, tert-butyl, pentyl, hexyl, heptyl or octyl;
- "alkoxy" is understood to mean an -O-alkyl radical, the alkyl group being as defined above. More particularly, the alkoxy radicals can be methoxy, ethoxy, propoxy or butoxy;
- "aryl" is understood to mean an aromatic cyclic group comprising between 5 and 12 carbon atoms, in particular a phenyl.

Preferably, the compound of thiopyridinone type according to the invention is of formula (I) or (I'), in which formulae:

R1 denotes a radical chosen from:

a) a hydrogen atom, or
b) a saturated linear $C_1$-$C_{10}$ or branched $C_3$-$C_{10}$ or unsaturated $C_2$-$C_{10}$ alkyl group, optionally substituted by one or more -OR3 groups; and

R2 denotes a radical chosen from:

a) a hydrogen atom,
b) a saturated linear $C_1$-$C_{10}$ or branched $C_3$-$C_{10}$ or cyclic $C_3$-$C_7$ alkyl group, optionally interrupted by one or more oxygen atoms and/or optionally substituted by one or more identical or different groups chosen from:

i) -OR3,
ii) -NR3R4,
iii) -CONHR3, or
iv) -COOR3,

c) a saturated $C_1$-$C_6$ alkyl group substituted by at least one phenyl radical optionally substituted by one or more hydroxyls and/or by one or more $C_1$-$C_3$ alkoxy radicals,
d) a phenyl group optionally substituted by one or more hydroxyls and/or by one or more $C_1$-$C_3$ alkoxy radicals,

R3 denoting a hydrogen atom or a saturated linear $C_1$-$C_5$ or branched $C_3$-$C_5$ hydrocarbon group;
R4 denoting a hydrogen atom or a saturated linear $C_1$-$C_5$ or branched $C_3$-$C_5$ hydrocarbon group;
and also their salts, their solvates, their optical isomers and their racemates.

According to one embodiment, the compound of thiopyridinone type according to the invention is of formula (I) or (I'), in which formulae:

R1 denotes a hydrogen atom or a saturated linear $C_1$-$C_6$ alkyl radical optionally substituted by one or more hydroxyl groups; and
R2 denotes a radical chosen from:

a) a hydrogen atom,
b) a saturated linear $C_1$-$C_{10}$ or branched $C_3$-$C_{10}$ or cyclic $C_5$-$C_7$ alkyl group, optionally interrupted by an oxygen atom and/or optionally substituted by a -$CONH_2$ group and/or optionally substituted by one or more hydroxyl groups,
c) a saturated $C_1$-$C_6$ alkyl group substituted by a phenyl radical optionally substituted by one or more hydroxyl or $C_1$-$C_3$ alkoxy radicals, or
d) a phenyl group,

and also their salts, their solvates, their optical isomers and their racemates.

According to this embodiment, the compound of thiopyridinone type according to the invention is preferably of formula (I) or (I'), in which formulae:

R1 denotes a hydrogen atom or a $C_1$-$C_6$ hydroxyalkyl group; and
R2 denotes:

a) a hydrogen atom,
b) a saturated linear $C_1$-$C_{10}$ or branched $C_3$-$C_{10}$ alkyl group, or
c) a saturated $C_1$-$C_6$ alkyl group substituted by a phenyl radical, in particular a benzyl group, and also their salts, their solvates, their optical isomers and their racemates.

More preferably still according to this embodiment, the compound of thiopyridinone type according to the invention is of formula (I) or (I'), in which formulae:

R1 denotes a hydrogen atom or a $C_1$-$C_6$ hydroxyalkyl group, preferably a hydrogen atom; and
R2 denotes a saturated linear $C_1$-$C_{10}$ or branched $C_3$-$C_{10}$ alkyl group, preferably a saturated linear $C_1$-$C_4$ alkyl group, in particular an ethyl.

According to another embodiment, the compound of thiopyridinone type according to the invention is of formula (I) or (I'), in which formulae:

R1 denotes a hydrogen atom or a saturated linear $C_1$-$C_6$ alkyl radical optionally substituted by one or more hydroxyl groups; and
R2 denotes a saturated linear $C_1$-$C_{10}$ or branched $C_3$-$C_{10}$ or cyclic $C_5$-$C_7$ alkyl group, optionally interrupted by an oxygen atom and optionally substituted by one or more -COOR3 groups,
R3 denoting a hydrogen atom or a saturated linear $C_1$-$C_6$ or branched $C_3$-$C_6$ or unsaturated $C_2$-$C_5$ alkyl group,
and also their salts, their solvates, their optical isomers and their racemates.

According to this embodiment, the thiopyridinone compound is more particularly of following formula (II) or (II'):

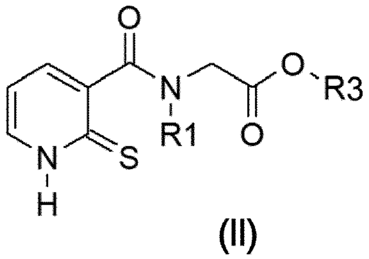

(II)

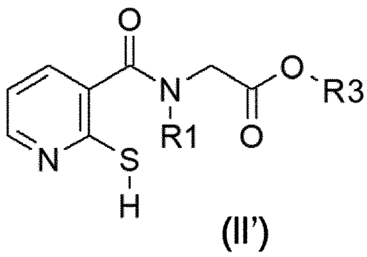

(II')

in which R1 and R3 are as defined above.

Preferably, according to this embodiment, the compound of thiopyridinone type is of formula (II) or (II'), in which formulae:

R1 denotes a radical chosen from:

a) a hydrogen atom, or
b) a saturated linear $C_1$-$C_4$ alkyl radical, preferably a methyl radical; and

R3 denotes a radical chosen from:

a) a hydrogen atom, or
b) a saturated linear $C_1$-$C_4$ alkyl group, preferably an ethyl group, or branched $C_3$-$C_4$ alkyl

group, preferably an isopropyl or isobutyl group,
and also their salts, their solvates, their optical isomers and their racemates.

More preferably still according to this embodiment, the compound of thiopyridinone type according to the invention is of formula (II) or (II'), in which:

R1 denotes a radical chosen from a hydrogen atom or a methyl, preferably a hydrogen atom; and
R3 denotes a radical chosen from a hydrogen atom or a saturated linear $C_1$-$C_4$ alkyl group, in particular an ethyl group, preferably a hydrogen atom.

More particularly preferred among the compounds of formulae (I) and (I') are the following compounds:

TABLE 1

| Compound No. | Structure | Chemical Name |
|---|---|---|
| 1 | 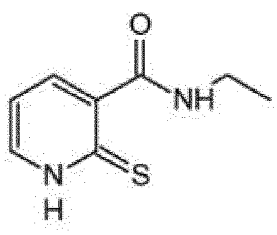 | N-ethy1-2-thioxo-1,2-dihydropyridine-3-carboxamide |
| 2 | 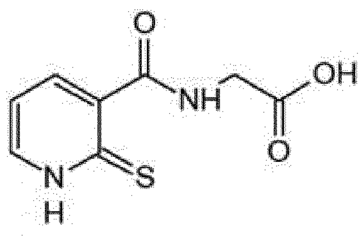 | N-[(2-thioxo-1,2-dihydropyridin-3-yl)carbonyl]glycine |

(continued)

| Compound No. | Structure | Chemical Name |
|---|---|---|
| 3 | 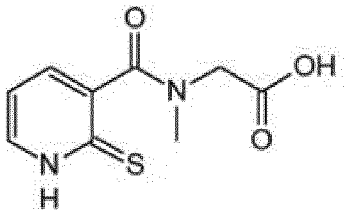 | N-methyl-N-[(2-thio,xo-1,2-dihydropyridin-3-yl)carbonyl] glycine |
| 4 | 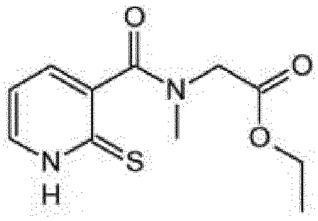 | Ethyl N-methyl-N-[(2-thioxo-1,2-dihydropyridin-3-yl) carbonyl] glycinate |
| 5 | 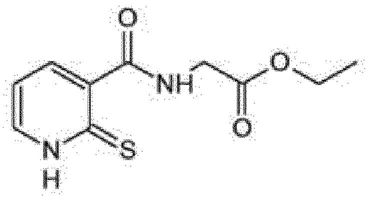 | Ethyl N-[(2-thioxo-1,2-dihydropyridin-3-yl)carbonyl] glycinate |

and also their salts, their solvates, their optical isomers and their racemates.

Among these compounds of formulae (I) and (I'), compound 2 is more particularly preferred, and also its salts, solvates and optical isomers.

The compounds of formula (I) or (I') can be obtained, in a known way, by reaction of 2-mercaptonicotinic acid and of an amine of formula (III):

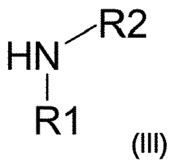

(III)

R1 and R2 having the meanings described above,
in particular in the presence of a coupling agent or of a base, for example carbonyldiimidazole.

The compounds of formula (I) or (I') can also be obtained, in a known way, by reaction of 2-mercaptonicotinic acid or of 2-chloronicotinic acid with an amine of formula (III), R1 and R2 having the meanings described above,

after having activated the carboxylic acid in the form of the compound of formula (IV):

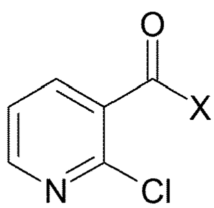

(IV)

where X forms an acid halide, a mixed anhydride, a carbamimidate or an acylphosphonate, by the activation of the carboxyl group of 2-chloronicotinic acid in the presence of an agent for the activation of carboxylic acids according to conventional methods for the activation of acids (which are described, for example, in Comprehensive Organic Transformations by R. Larock, published by Wiley VCH, in the chapter Interconversion of Nitriles, Carboxylic Acids and Derivatives).

Use is preferably made of an agent for the activation of carboxylic acids which makes it possible to form an acid chloride (for example by use of thionyl or oxalyl chloride, or of 1-chloro-N,N,2-trimethyl-1-propenamine) or to form a mixed anhydride (using alkyl chloroformates) or use of carbodiimides or diethyl cyanophosphate to form carbamimidates or acylphosphonates (Phosphorus in organic synthesis - XI, Amino acids and peptides -XXI, Reaction of diethyl phosphorocyanidate with carboxylic acids. A new synthesis of carboxylic esters and amides, Tetrahedron, 32, 1976, 2211-2217).

When 2-chloronicotinic acid is used as starting reactant, the chloroamide obtained is subsequently used in a reaction for exchange between chlorine and sulfur by means of reactants, in particular NaSH, thiourea, sodium thiosulfate or thioacetic acid (in a basic medium).

As mentioned above, according to one embodiment of the invention, the compound of thiopyridinone type can be of formula (II) or (II') as are defined above.

It is understood that the compounds of formula (II) or (II') can be obtained by the same process as that described in detail above, the amine involved being more specifically of formula (V):

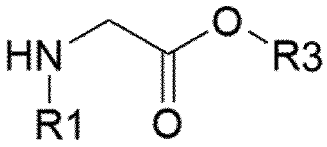

(V)

in which R1 and R3 are as defined above.

The synthesis scheme below summarizes the preparation of compounds of formula (II) or (II') according to this embodiment, the compound (VI) denoting the chloroamide intermediate:

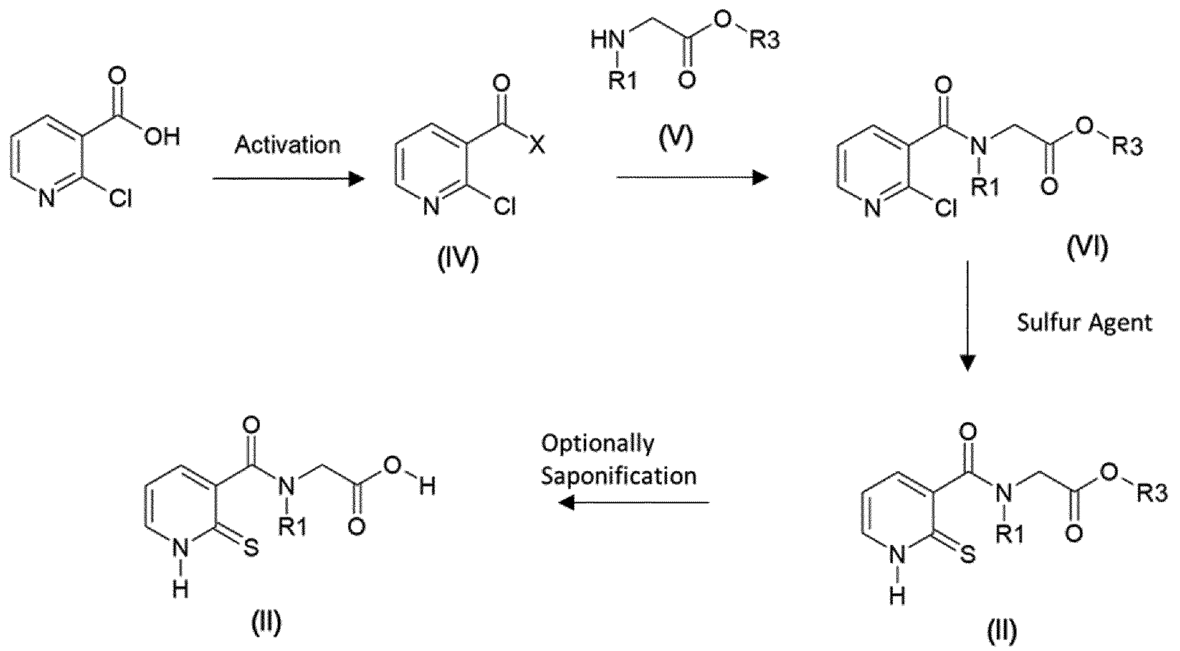


Compounds of formula (I) or (I') are in particular described in the following documents: EP-A-298752 , WO03/014062, EP-A-298752 , FR-A-2349591, EP-A-2555450 , WO 03/014062, WO 2008/012532, WO 2012/080075A1 and WO 2017/102349A1, and in the publications:

- A. Monge and V. Martinez-Merino; Synthesis of 2-substituted 3-Oxoisothiazolo[5,4-b]pyridines; J. Heterocyclic. Chem., 22, 1353 (1985);
- A. Dunn and R. Norrie; Synthesis of pyrido-1,3-thiazines; Zeitschrift fur Chemie, 1988, Vol. 28, No. 6, pp 212/214;
- S. Andreae; J. Prakt. Chem., 339 (1997), 152-158;
- S. Gorsuch; Biorganic & Medicinal Chemistry, 17 (2009), 467-474;
- A. Monge et al.; J. Heterocyclic Chem., 25, 23 (1988); and
- M. Pregnolato; Il Farmaco, 55 (2000), 669-679.

## COMPOSITION

According to one aspect of the invention, the compound(s) of thiopyridinone type can be incorporated in a cosmetic composition intended for a topical application, in order to prevent the formation of cutaneous blackheads, in particular in order to inhibit the development of the black or brown colour of the comedones.

Thus, the present invention is targeted at a cosmetic use in which at least one compound of thiopyridinone type employed according to the invention is present in a cosmetic composition intended to prevent the black or brown colouration of the comedones.

Advantageously, in this cosmetic use, said compound of thiopyridinone type is present in the cosmetic composition in a content ranging from 0.1% to 5.0% by weight, preferably from 0.1% to 3.0% by weight, in particular from 0.5% to 3.0% by weight, with respect to the total weight of the cosmetic composition.

Preferably, a compound of thiopyridinone type employed according to the invention, when it is present within a composition, can be formulated in a physiologically acceptable medium. Such a medium is considered to be physiologically acceptable when it does not cause any tingling, tautness or discomfort unacceptable for the user.

A composition used according to the invention can be provided in any formulation form normally used in the cosmetics field.

It can in particular be in the form of an aqueous or aqueous/alcoholic solution, which is optionally gelled, of a dispersion of the lotion type, which is optionally a two-phase lotion, of an oil-in-water or water-in-oil or multiple emulsion, of an aqueous gel, of a dispersion of oils in an aqueous phase, in particular by means of spherules, it being possible for these spherules to be polymer particles or, better still, lipid vesicles of ionic and/or non-ionic type, or else in the form of a powder, of a serum, of a paste or of a flexible rod or also of a stick. It can be of solid, pasty or more or less fluid liquid consistency.

Thus, the composition can comprise any constituent normally employed in the topical application and administration envisaged.

Mention may in particular be made of water, solvents, compounds forming a fatty phase which is liquid at ambient temperature, for example oils of mineral, animal and/or vegetable origin, waxes, pigments, fillers, surfactants, thickeners, gelling agents, preservatives and their mixtures in all proportions.

A composition according to the invention can advantageously be provided in the form of an emulsion, in particular obtained by dispersion of an aqueous phase in a fatty phase (W/O) or of a fatty phase in an aqueous phase (O/W), of liquid or semi-liquid consistency of the milk type, or of soft, semi-solid or solid consistency of the cream or gel type, or also of multiple emulsion (W/O/W or O/W/O). These compositions are prepared according to the usual methods.

A composition used according to the invention can advantageously comprise at least one fatty phase which is liquid at ambient temperature and atmospheric pressure.

Such a fatty phase can comprise at least one non-volatile ether oil having from 10 to 40 carbon atoms and/or at least one non-volatile monoester oil.

Within the meaning of the present invention, the term "*non-volatile oil*" is understood to denote an oil, the vapour pressure of which at ambient temperature and atmospheric pressure is non-zero and less than $10^{-3}$ mmHg (0.13 Pa).

Mention may in particular be made, as example of ethers having from 10 to 40 carbon atoms, of dicaprylyl ether (CTFA name: Dicaprylyl ether).

Mention may in particular be made, as preferred monoesters, of isononyl isononanoate, oleyl erucate and/or 2-octyldodecyl neopentanoate, preferably isononyl isononanoate.

The amount of fatty phase which is liquid at ambient temperature and atmospheric pressure present in the compositions according to the invention can range, for example, from 0.01% to 50% by weight and preferably from 0.1% to 30% by weight, with respect to the total weight of the composition.

The emulsions according to the invention can comprise at least one emulsifier chosen from amphoteric, anionic, cationic or non-ionic emulsifiers, used alone or as a mixture.

Advantageously, the emulsifiers are appropriately chosen according to the emulsion to be obtained (W/O or O/W). The emulsifiers are generally present in the composition in a proportion which can range from 0.3% to 30% by weight and preferably from 0.5% to 20% by weight, with respect to the total weight of the composition.

Mention may be made, for the O/W emulsions, for example, as emulsifiers, of non-ionic surfactants, and in

particular esters of polyols and of fatty acids having a saturated or unsaturated chain comprising, for example, from 8 to 24 carbon atoms and better still from 12 to 22 carbon atoms, and their oxyalkylenated derivatives, that is to say derivatives comprising oxyethylene and/or oxypropylene units, for example the esters of glycerol and of $C_8$-$C_{24}$ fatty acids, and their oxyalkylenated derivatives; the esters of polyethylene glycol and of $C_8$-$C_{24}$ fatty acids, and their oxyalkylenated derivatives; the esters of sorbitol and of $C_8$-$C_{24}$ fatty acids, and their oxyalkylenated derivatives; the esters of sugar (sucrose, glucose or alkylglucose) and
of $C_8$-$C_{24}$ fatty acids, and their oxyalkylenated derivatives; the ethers of fatty alcohols; the ethers of sugar and of $C_8$-$C_{24}$ fatty alcohols, and their mixtures in all proportions.

A composition according to the invention can additionally comprise at least one silicone elastomer, in particular the products sold under the KSG names by Shin-Etsu, under the Trefil, BY29 or EPSX names by Dow Corning or under the Gransil names by Grant Industries.

A composition used according to the invention can additionally contain one or more adjuvants commonly used in the cosmetics field, in particular sequestering agents, odour absorbers, UV-screening agents, fragrances, mattifying agents, and abrasive fillers or exfoliating agents, and their mixtures in all proportions.

A composition used according to the invention can advantageously comprise at least one additional active principle.

It can in particular be at least one active principle for caring for greasy skin.

It can also advantageously comprise at least one additional active principle for caring for aged skin.

The expression "additional active principle" is understood to mean, in the context of the present invention, a compound which has, by itself, that is to say not requiring the intervention of an external agent in order to activate it, a biological activity which can in particular be:

- a desquamating activity (which makes possible the opening of the comedones), and/or
- an antimicrobial activity (in particular with regard to *P. acnes*), and/or
- a soothing or anti-irritant activity, and/or
- a sebum-regulating or anti-seborrhoeic activity, and/or
- an astringent activity, and/or
- an activity intended to combat and/or prevent the signs of ageing, in particular chronobiological ageing, for example wrinkles and fine lines.

The additional active principle which can be used in the compositions of the invention is preferentially chosen from desquamating agents, soothing agents, anti-irritant agents, sebum-regulating or anti-seborrhoeic agents, astringent agents and their mixtures in all proportions.

The additional active principle for caring for greasy skin used in the composition according to the invention can represent from 0.0001% to 20%, preferably from 0.01% to 10% and better still from 0.01% to 5% by weight, with respect to the total weight of the composition. Mention may in particular be made, as example of such an additional active principle, of salicylic acid and pro-xylane, in particular extracted from rye seeds.

Moreover, a composition employed according to the invention can advantageously comprise from 5% to 80% by weight and preferably from 35% to 75% by weight of water, with respect to the total weight of said composition.

The pH of said composition is advantageously less than or equal to 7, preferably ranging from 5 to 6.5, limits included.

A composition according to the invention can additionally comprise retinol, otherwise known as vitamin A, preferably all-trans-retinol.

More particularly, a composition according to the invention can comprise an amount of retinol ranging from 0.02% to 5.0% by weight, in particular from 0.05% to 3.0% by weight, with respect to the total weight of the composition.

Thus, a composition employed according to the invention can comprise at least one additional active principle, in particular chosen from salicylic acid, pro-xylane, retinol and their mixtures.

A composition according to the invention can additionally comprise at least one polyol, namely an organic molecule comprising at least two free hydroxyl groups, in particular chosen from ethylene glycol, propylene glycol, 1,3-propanediol, 1,3-butylene glycol, isoprene glycol, 1,2-pentylene glycol, hexylene glycol, caprylyl glycol, dipropylene glycol, glycerol, pentaerythritol, trimethylolpropane, polyglycerols, polyethylene glycols and their mixtures.

A composition according to the invention can additionally comprise at least one gelling agent, in particular a hydrophilic gelling agent, namely a compound capable of gelling the aqueous phase of the compositions according to the invention. Mention may be made, as examples of hydrophilic gelling agents, of the polymers and copolymers of 2-acrylamido-2-methylpropanesulfonic acid, which are crosslinked and/or neutralized, polysaccharides and their mixtures.

A composition according to the invention can also comprise at least one derivative of polyethylene glycol and of mono-, di- and triglycerides of an acid comprising at least one alkyl chain ranging from $C_6$ to $C_{16}$, and having at least two ethylene oxide groups, in particular chosen from derivatives of polyethylene glycol and of mono-, di- and triglycerides

of caprylic acid and of capric acid, for example that comprising 6 ethylene oxide groups (INCI name: PEG-6 caprylic/capric glycerides), sold under the name Softigen 767 by Sasol. A composition according to the invention can also comprise at least one colourant chosen, for example, from pigments, pearlescent agents, dyes, effect materials and their mixtures in all proportions.

These colourants can be present in a content ranging from 0.01% to 50% by weight and preferably from 0.01% to 30% by weight, with respect to the total weight of the composition. A composition according to the invention can additionally comprise at least one filler, in particular in a content ranging from 0.01% to 50% by weight, preferably ranging from 0.01% to 30% by weight, with respect to the total weight of the composition.

These fillers can be inorganic or organic and of any shape, platelet, spherical or oblong, whatever the crystallographic form (for example sheet, cubic, hexagonal, orthorhombic or amorphous).

Of course, a person skilled in the art will take care to choose the optional additional compound(s), and/or their amount, in such a way that the advantageous properties of the thiopyridinone compounds according to the invention are not, or not substantially, detrimentally affected by the envisaged addition and that the properties of the compositions resulting therefrom are compatible with the topical administration route.

A composition of the invention can advantageously exhibit a firm and compact feel when taken up. It can be thick on application and subsequently be transformed, melt and release freshness.

According to another alternative form, a composition of the invention can advantageously be provided in the form of a more or less wide stick, such as a pencil, for example.

A composition can alternatively have the form of a product for caring for or making up the face and/or the body, and be packaged, for example, in the form of cream in a jar or of fluid in a tube or as a pump-action spray.

A composition according to the invention can be manufactured by any known process generally used in the cosmetics field.

The ingredients are mixed before they are shaped, in the order and under conditions which are easily determined by a person skilled in the art.

According to a specific form of the invention, other agents intended to make the appearance and/or the texture of the skin more attractive can also be added to the composition according to the invention.

## COSMETIC METHODS

As mentioned above, the present invention relates to a cosmetic method for preventing the black or brown colouration of the comedones, comprising the topical application, to the skin, of a cosmetic composition comprising at least one compound corresponding to the formula (I) or (I') as are defined above.

The cosmetic method of the invention is carried out by administering, by the topical route, a composition comprising at least one compound of thiopyridinone type as defined according to the invention, preferably in a physiologically acceptable medium.

The topical administration consists of the external application to the skin of cosmetic compositions according to the usual techniques for use of these compositions.

By way of illustration, the cosmetic method according to the invention can be carried out by topical application, for example daily, of at least one compound of thiopyridinone type in accordance with the invention, which can, for example, be formulated in the form of a cream, gel, serum, lotion, emulsion, make-up-removing milk, stick or aftersun composition.

According to one embodiment, the application is repeated, for example 2 to 3 times daily for one day or more and generally over an extended period of at least 4 weeks, indeed even 4 to 15 weeks, with, if appropriate, one or more periods of interruption.

According to another embodiment, the application is daily (once per day) and generally over an extended period of at least 4 weeks, indeed even 4 to 15 weeks, with, if appropriate, one or more periods of interruption.

According to another embodiment, the composition including at least one compound of thiopyridinone type as defined above will be applied to skin regions which have been cleansed beforehand, for example using an appropriate soap, and/or freed beforehand from at least one blackhead, for example using an adhesive patch or a mechanical action. Furthermore, treatment combinations with optionally topical forms, in order to complement or to reinforce the activity of the compound of thiopyridinone type as defined by the invention, can be envisaged.

It might be possible to imagine a topical treatment with a composition containing at least one compound of thiopyridinone type in accordance with the invention, combined with a supplementary composition dedicated to a topical application and containing at least one active principle distinct from a compound of thiopyridinone type as required according to the invention.

Thus, the present invention also relates to a cosmetic method for preventing the black or brown colouration of the comedones, comprising:

a) a stage of topical application, to the skin, of a cosmetic composition comprising at least one compound of thiopyridinone type as defined according to the invention; and

b) a stage of topical application of a composition different from the composition applied in stage a) and containing at least one active principle distinct from the compound of thiopyridinone type defined according to the invention.

Such a composition applied in stage b) can be described as "supplementary composition". Stages a) and b) defined above can be carried out simultaneously or successively.

This supplementary composition, which is different from the composition employed according to the invention, and applicable by the topical route, can in particular be in the form of an emulsified oil-in-water gel, comprising retinol; at least one polyol; at least one hydrophilic gelling agent; at least one derivative of polyethylene glycol and of mono-, di- and triglycerides of an acid comprising at least one alkyl chain ranging from $C_6$ to $C_{16}$, and having at least two ethylene oxide groups; at least one non-volatile ether oil having from 10 to 40 carbon atoms; and at least one non-volatile monoester oil.

Throughout the description, including the claims, the expressions "between ... and ...", "of between ... and ..." and "ranging from ... to ..." should be understood as meaning limits included, unless otherwise specified.

The examples which follow illustrate the present invention without limiting the scope thereof.

## Examples

### *Methodology on reconstituted skin used for the characterization of an appearance of dark-coloured structures and its development over time*

Within the meaning of the present invention, the term "dark-coloured structures" is understood to denote the structures responsible for the colouration of blackheads.

This method is targeted at characterizing the change over time in the expression of colour, brought about by an intracellular extract of bacteria of *Cutibacterium acnes* type (formally listed under the name *Propionibacterium acnes*) in the presence of 3,4-dihydroxy-L-phenylalanine (L-DOPA), which extract is deposited at the surface of reconstituted skin. To do this, an intracellular extract of bacteria of *Cutibacterium acnes* CIP 53.117 type, stored at 4°C in lyophilized form, is suspended in 1X PBS (Dulbecco's Phosphate Buffered Saline, supplied by Gibco BRL Life Technologies) at a concentration of 6 mg/ml. This extract is subsequently deposited at the surface of an EpiSkin epidermis in the presence of 3,4-dihydroxy-L-phenylalanine (L-DOPA - supplied by Sigma-Aldrich) at 1 mg/ml.

The expression of a colouring effect and its development towards a dark colour are observed visually, starting from 48 hours of incubation.

### *In vitro methodology used for the evaluation of the ability to inhibit the development of the colour*

This method is based on the measurement of absorbance, over time, carried out directly on a composition formed of a mixture of intracellular extract of bacteria of *Cutibacterium acnes* type, of 3,4-dihydroxy-L-phenylalanine (L-DOPA) and of a test compound.

The measurements are compared with those carried out for a mixture of an intracellular extract of bacteria of *Cutibacterium acnes* type and of 3,4-dihydroxy-L-phenylalanine (L-DOPA), in the absence of test compound (reference).

The method is adapted from a method for the enzymatic assay of the tyrosinase activity (Behbahani et al., Microchemical Journal (1993), 47, 251), which is based on the absorbance properties at 475 nm of a coloured intermediate of the pathway for the biosynthesis of melanin starting from L-DOPA, namely dopachrome, a product of the oxidation of L-DOPA.

### Preparation of the reference composition

An intracellular extract of bacteria of *Cutibacterium acnes* CIP 53.117 type stored at 4°C in the lyophilized form is suspended in 1X PBS (supplied by Gibco BRL Life Technologies) at a concentration of 6 mg/ml.

A solution of L-DOPA at a concentration of 0.5 mg/ml is prepared by bringing together L-DOPA (supplied by Sigma-Aldrich) and milliQ water, so as to have an L-DOPA concentration of 1 mg/ml, then diluting with 1X PBS (Dulbecco's Phosphate Buffered Saline, supplied by Gibco BRL Life Technologies).

The mixture of 50 $\mu$l of these two solutions and of 100 $\mu$l of 1X PBS (Dulbecco's Phosphate Buffered Saline, supplied by Gibco BRL Life Technologies) results in a composition with a total volume of 200 $\mu$l containing L-DOPA (at a concentration of 125 $\mu$g/ml) and *Cutibacterium acnes* extract (at a concentration of 1.5 mg/ml).

**Preparation of a composition 1 comprising only a *Cutibacterium acnes* extract**

The abovementioned 6 mg/ml *Cutibacterium acnes* extract was diluted with 1X PBS (Dulbecco's Phosphate Buffered Saline, supplied by Gibco BRL Life Technologies) so as to form a composition of 200 μl comprising a concentration of 1.5 mg/ml of *Cutibacterium acnes.*

**Preparation of a composition 2 comprising only L-DOPA**

The abovementioned 0.5 mg/ml L-DOPA solution was diluted with 1X PBS (Dulbecco's Phosphate Buffered Saline, supplied by Gibco BRL Life Technologies) so as to form a composition of 200 μl comprising a concentration of 125 μg/ml of L-DOPA.

**Absorbance measurement**

The test compositions and the reference composition are introduced separately into wells of a 96-well microplate suitable for reading in visible spectrophotometry, and placed at a temperature of 32°C.

The formation of a coloured product is monitored over time by spectrophotometry. To do this, the absorbance, or optical density (O.D.), is measured at 475 nm from the time 0 to the time 2 hours by means of a Spectra Max M5e reader and the Spectra Max software (the time 0 corresponds to the introduction of the composition into the wells).

**Confirmation of the method**

The graph of figure 1 brings out the formation of a coloured product for the reference composition, the product of the oxidation of L-DOPA.

For compositions 1 et 2 respectively comprising a *Cutibacterium acnes* extract alone and L-DOPA alone, no significant increase in the absorbance over time is observed.

This confirms the catalytic activity of the bacterial extract of *Cutibacterium acnes* on the development of the dark colour by oxidation of L-DOPA.

It is thus possible to monitor the formation of the coloured intermediate of the pathway for the biosynthesis of melanin by virtue of its properties of absorption at 475 nm. Consequently, the effectiveness of a compound in preventing the development of the colour of the cutaneous blackheads can be evaluated by this means.

***Parameters studied for the evaluation of the effectiveness of the various test compounds***

The ability to inhibit the development of the colour of a given compound was assessed by comparing the absorbance measurements obtained for the test compositions with those obtained for the reference composition.

More specifically, the inhibitory effect of the test compound was evaluated by comparing the initial rate of oxidation of the L-DOPA under the action of the bacterial extract at 475 nm for the test composition with that of the reference composition in order to obtain a corresponding percentage of inhibition.

In practice, the initial rate (Vi) of the oxidation reaction is calculated from the linear part of the curve of the graph of the absorbance values. As this part lies between 10 and 40 minutes (see the graph in Figure 1), the initial rate of the oxidation reaction is determined using the following relationship:

$$Vi = \frac{O.D.measured\ at\ 40\ minutes - O.D.measured\ at\ 10\ minutes}{30\ minutes}$$

The percentage of inhibition (%inhib) of a given compound with regard to the formation of the coloured product is subsequently obtained using the values of the initial rate of the oxidation reaction for the reference composition ($Vi_{ref}$) and for the test composition ($Vi_{test}$) according to the following relationship:

$$\%inhib = \frac{Vi_{ref} - Vi_{test}}{Vi_{ref}} \times 100$$

Within the meaning of the invention, a compound is regarded as having an inhibitory effect if the %inhib value is at least 15%.

**Example 1 - Characterization of the effect of the *Cutibacterium acnes* bacteria (formerly listed under the name *Propionibacterium acnes*) on the appearance of dark-coloured structures.**

The production of dark-coloured structures from L-DOPA by the bacterium *Cutibacterium acnes* found in the comedones was demonstrated in accordance with the protocol defined above.

This is because, starting from 48 hours of incubation at 32°C, the formation of dark-coloured structures in the form of grains was observed.

Figure 2 demonstrates this formation. It is a photograph of the surface of the epidermis magnified 7 times using a stereoscopic microscope. This image was taken after 120 hours of incubation, and shows that bacteria of *Cutibacterium acnes* type are capable of producing pigmented structures.

It has thus been shown that the species *Cutibacterium acnes* exhibits an (enzymatic and/or chemical) catalytic system for the synthesis of dark-coloured structures.

**Example 2 - *In vitro* evaluation of the ability of compound 1 in accordance with the invention to inhibit the development of the colour**

4 test compositions 11, I2, I3 and I4 comprising compound 1, in accordance with the invention, at respective concentrations of 12.5, 25, 50 and 100 μM, were prepared. These compositions, with a total volume of 200 μl, also contain L-DOPA (25 μg), the *Cutibacterium acnes* extract (0.3 mg) and the PBS buffer which are mentioned in the methodology part above. The change in the optical density at 475 nm of these four compositions, and also of the reference composition, is then measured according to the protocol indicated in the methodology part which appears above.

The measured optical density values are collated in the following Table 2.

TABLE 2

| Duration [Minutes] | 0 | 10 | 20 | 30 | 40 | 60 | 80 | 100 | 120 |
|---|---|---|---|---|---|---|---|---|---|
| Reference (*without compound 1*) | 0.0538 | 0.0553 | 0.0578 | 0.0611 | 0.0644 | 0.0691 | 0.0714 | 0.0727 | 0.0726 |
| I1 | 0.0553 | 0.0553 | 0.0561 | 0.0569 | 0.0584 | 0.0605 | 0.0631 | 0.0661 | 0.0681 |
| I2 | 0.0554 | 0.0560 | 0.0572 | 0.0587 | 0.0596 | 0.0601 | 0.0611 | 0.0627 | 0.0640 |
| I3 | 0.0541 | 0.0565 | 0.0568 | 0.0569 | 0.0568 | 0.0575 | 0.0574 | 0.0598 | 0.0597 |
| I4 | 0.0568 | 0.0555 | 0.0548 | 0.0561 | 0.0555 | 0.0557 | 0.0559 | 0.0564 | 0.0567 |

The percentage of inhibition values, calculated from the mathematical formulae described in detail in the methodology part above, are presented in Table 3 below.

TABLE 3

| Composition | I1 | I2 | I3 | I4 |
|---|---|---|---|---|
| %inhib | 66 % | 60 % | 96 % | 100 % |

Compound 1 in accordance with the invention is thus very effective in inhibiting the development of the colour which is observed in the absence of treatment (Reference).

**Example 3 - *In vitro* evaluation of the ability of compound 2 in accordance with the invention to inhibit the development of the colour**

1 test composition 15 comprising compound 2, in accordance with the invention, at a concentration de 100 μM, was prepared. This composition, with a total volume of 200 μl, also contains L-DOPA (25 μg), the *Cutibacterium acnes* extract (0.3 mg) and the PBS buffer which are mentioned in the methodology part above. The change in the optical density at 475 nm of this composition, and also of the reference composition, is then measured according to the protocol indicated in the methodology part which appears above.

The measured optical density values are collated in the following Table 4.

TABLE 4

| Duration [Minutes] | 0 | 10 | 20 | 30 | 40 | 60 | 80 | 100 | 120 |
|---|---|---|---|---|---|---|---|---|---|
| Reference (*without compound 2*) | 0.0554 | 0.0571 | 0.0591 | 0.0620 | 0.0652 | 0.0695 | 0.0723 | 0.0729 | 0.0750 |
| I5 | 0.0547 | 0.0561 | 0.0567 | 0.0569 | 0.0574 | 0.0578 | 0.0578 | 0.0578 | 0.0580 |

The percentage of inhibition value, calculated from the mathematical formulae described in detail in the methodology part above, is 84%.

Compound 2 in accordance with the invention is thus very effective in inhibiting the development of the colour which is observed in the absence of treatment (Reference).

**Example 4 - *In vitro* evaluation of the ability of compound 3 in accordance with the invention to inhibit the development of the colour**

4 test compositions I6, I7, I8 and I9 comprising compound 3, in accordance with the invention, at respective concentrations of 12.5, 25, 50 and 100 μM, were prepared. These compositions, with a total volume of 200 μl, also contain L-DOPA (25 μg), the *Cutibacterium acnes* extract (0.3 mg) and the PBS buffer which are mentioned in the methodology part above. The change in the optical density at 475 nm of these four compositions, and also of the reference composition, is then measured according to the protocol indicated in the methodology part which appears above.

The measured optical density values are collated in the following Table 5.

TABLE 5

| Duration [Minutes] | 0 | 10 | 20 | 30 | 40 | 60 | 80 | 100 | 120 |
|---|---|---|---|---|---|---|---|---|---|
| Reference (*without compound 3*) | 0.0540 | 0.0574 | 0.0595 | 0.0622 | 0.0652 | 0.0697 | 0.0722 | 0.0729 | 0.0730 |
| I6 | 0.0555 | 0.0565 | 0.0575 | 0.0595 | 0.0613 | 0.0647 | 0.0677 | 0.0686 | 0.0694 |
| I7 | 0.0549 | 0.0569 | 0.0578 | 0.0594 | 0.0607 | 0.0630 | 0.0651 | 0.0664 | 0.0677 |
| I8 | 0.0539 | 0.0561 | 0.0575 | 0.0587 | 0.0594 | 0.0609 | 0.0623 | 0.0632 | 0.0639 |
| I9 | 0.0579 | 0.0606 | 0.0612 | 0.0619 | 0.0623 | 0.0618 | 0.0606 | 0.0605 | 0.0609 |

The percentage of inhibition values, calculated from the mathematical formulae described in detail in the methodology part above, are presented in Table 6 below.

TABLE 6

| Composition | I6 | I7 | I8 | I9 |
|---|---|---|---|---|
| %inhib | 39% | 51% | 58% | 78% |

Compound 3 in accordance with the invention is thus very effective in inhibiting the development of the colour which is observed in the absence of treatment (Reference).

**Example 5 - *In vitro* evaluation of the ability of compound 4 in accordance with the invention to inhibit the development of the colour**

4 test compositions 110, 111, 112 and 113 comprising compound 4, in accordance with the invention, at respective concentrations of 12.5, 25, 50 and 100 μM, were prepared. These compositions, with a total volume of 200 μl, also contain L-DOPA (25 μg), the *Cutibacterium acnes* extract (0.3 mg) and the PBS buffer which are mentioned in the methodology part above. The change in the optical density at 475 nm of these four compositions, and also of the reference composition, is then measured according to the protocol indicated in the methodology part which appears above.

The measured optical density values are collated in the following Table 7.

TABLE 7

| Duration [Minutes] | 0 | 10 | 20 | 30 | 40 | 60 | 80 | 100 | 120 |
|---|---|---|---|---|---|---|---|---|---|
| Référence (*without compound 4*) | 0.0562 | 0.0572 | 0.0598 | 0.0635 | 0.0671 | 0.0717 | 0.0741 | 0.0749 | 0.0751 |
| I10 | 0.0550 | 0.0571 | 0.0585 | 0.0604 | 0.0627 | 0.0659 | 0.0679 | 0.0696 | 0.0709 |
| I11 | 0.0573 | 0.0586 | 0.0595 | 0.0609 | 0.0623 | 0.0650 | 0.0682 | 0.0695 | 0.0703 |
| I12 | 0.0558 | 0.0574 | 0.0580 | 0.0591 | 0.0599 | 0.0617 | 0.0634 | 0.0654 | 0.0666 |
| I13 | 0.0578 | 0.0579 | 0.0593 | 0.0601 | 0.0608 | 0.0618 | 0.0623 | 0.0627 | 0.0631 |

The percentage of inhibition values, calculated from the mathematical formulae described in detail in the methodology part above, are presented in Table 8 below.

TABLE 8

| Composition | I10 | I11 | I12 | I13 |
|---|---|---|---|---|
| %inhib | 43 % | 63 % | 75 % | 71% |

Compound 4 in accordance with the invention is thus very effective in inhibiting the development of the colour which is observed in the absence of treatment (Reference).

**Example 6 - *In vitro* evaluation of the ability of compound 5 in accordance with the invention to inhibit the development of the colour**

4 test compositions 114, 115, 116 and 117 comprising compound 5, in accordance with the invention, at respective concentrations of 12.5, 25, 50 and 100 μM, were prepared. These compositions, with a total volume of 200 μl, also contain L-DOPA (25 μg), the *Cutibacterium acnes* extract (0.3 mg) and the PBS buffer which are mentioned in the methodology part above. The change in the optical density at 475 nm of these four compositions, and also of the reference composition, is then measured according to the protocol indicated in the methodology part which appears above.

The measured optical density values are collated in the following Table 9.

TABLE 9

| Duration [Minutes] | 0 | 10 | 20 | 30 | 40 | 60 | 80 | 100 | 120 |
|---|---|---|---|---|---|---|---|---|---|
| Reference (*without compound 5*) | 0.0554 | 0.0563 | 0.0587 | 0.0624 | 0.0649 | 0.0695 | 0.0722 | 0.0732 | 0.0736 |
| I14 | 0.0531 | 0.0535 | 0.0550 | 0.0559 | 0.0572 | 0.0601 | 0.0636 | 0.0659 | 0.0674 |
| I15 | 0.0559 | 0.0567 | 0.0572 | 0.0575 | 0.0578 | 0.0588 | 0.0602 | 0.0627 | 0.0651 |
| I16 | 0.0539 | 0.0554 | 0.0557 | 0.0560 | 0.0565 | 0.0573 | 0.0579 | 0.0585 | 0.0593 |
| I17 | 0.0532 | 0.0534 | 0.0548 | 0.0550 | 0.0550 | 0.0555 | 0.0557 | 0.0561 | 0.0563 |

The percentage of inhibition values, calculated using the mathematical formulae described in detail in the methodology part above, are presented in Table 10 below.

TABLE 10

| Composition | I14 | I15 | I16 | I17 |
|---|---|---|---|---|
| %inhib | 57% | 87% | 87% | 82% |

Compound 5 in accordance with the invention is thus very effective in inhibiting the development of the colour which is observed in the absence of treatment (Reference).

**Example 7 - *In vitro* evaluation of the ability of a compound not in accordance with the invention, lucinol, to inhibit the development of the colour**

One test composition, C18, comprising lucinol at a respective concentration de 100 μM, was prepared. This composition, with a total volume of 200 μl, also contains L-DOPA (25 μg), the *Cutibacterium acnes* extract (0.3 mg) and the PBS buffer which are mentioned in the methodology part above. The change in the optical density at 475 nm of this composition, and also of the reference composition, is then measured according to the protocol indicated in the methodology part which appears above.

The measured optical density values are collated in the following Table 11.

TABLE 11

| Duration [Minutes] | 0 | 10 | 20 | 30 | 40 | 60 | 80 | 100 | 120 |
|---|---|---|---|---|---|---|---|---|---|
| Reference (*without lucinol*) | 0.0593 | 0.0598 | 0.0627 | 0.0667 | 0.0706 | 0.0766 | 0.0800 | 0.0815 | 0.0816 |
| C18 | 0.0552 | 0.0563 | 0.0591 | 0.0629 | 0.0671 | 0.0730 | 0.0758 | 0.0768 | 0.0765 |

The percentage of inhibition value, calculated using the mathematical formulae described in detail in the methodology part above, is 0%.

Lucinol, not in accordance with the invention, does not make it possible to inhibit the development of the colour which is observed in the absence of treatment (Reference).

**Example *8 - In vitro* evaluation of the ability of a compound not in accordance with the invention, kojic acid, to inhibit the development of the colour**

One test composition C19 comprising kojic acid, sold under the reference K3125 by Sigma-Aldrich, at a concentration of 100 μM, was prepared. This composition, with a total volume of 200 μl, also contains L-DOPA (25 μg), the *Cutibacterium acnes* extract (0.3 mg) and the PBS buffer which are mentioned in the methodology part above. The change in the optical density at 475 nm of this composition, and also of the reference composition, is then measured according to the protocol indicated in the methodology part which appears above. The measured optical density values are collated in the following Table 12.

TABLE 12

| Duration [Minutes] | 0 | 10 | 20 | 30 | 40 | 60 | 80 | 100 | 120 |
|---|---|---|---|---|---|---|---|---|---|
| Reference (*without kojic acid*) | 0.0608 | 0.0619 | 0.0659 | 0.0703 | 0.0744 | 0.0805 | 0.0835 | 0.0845 | 0.0843 |
| C19 | 0.0599 | 0.0614 | 0.0646 | 0.0698 | 0.0741 | 0.0804 | 0.0837 | 0.0845 | 0.0843 |

The percentage of inhibition value, calculated using the mathematical formulae described in detail in the methodology part above, is -1%.

Kojic acid, not in accordance with the invention, does not make it possible to inhibit the development of the colour which is observed in the absence of treatment (Reference).

**Example 9 - Cosmetic compositions comprising at least one compound of thiopyridinone type in accordance with the invention**

A composition for topical application is prepared which comprises:

TABLE 13

| | |
|---|---|
| Ethyl N-methyl-N-[(2-thioxo-1,2-dihydropyridin-3-yl)carbonyl] glycinate | 2 g |
| PEG400 | 68 g |
| Ethanol | 30 g |

The composition applied to the skin makes it possible to prevent the black or brown colouration of the comedones.

A composition in the form of a gel for topical application is prepared which comprises:

TABLE 14

| | |
|---|---|
| N-[(2-thioxo-1,2-dihydropyridin-3-yl)carbonyl]glycine | 0.5 % by weight |
| Carbomer (Carbopol® 981 from Lubrizol) | 1.0 % by weight |
| Preservative | q.s. |
| Water | q.s. for 100% |

The composition applied to the skin makes it possible to prevent the black or brown colouration of the comedones.

## Claims

1. Cosmetic use of at least one compound corresponding to the following formula (I) or (I'):

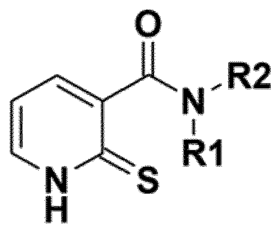
(I)

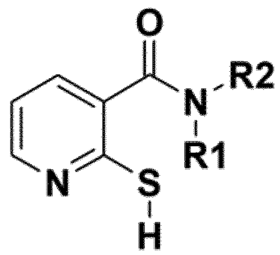
(I')

in which formulae:

R1 and R2, which are identical or different, denote a radical chosen from:

a) a hydrogen atom;
b) a saturated linear $C_1$-$C_{20}$ or branched $C_3$-$C_{20}$ or unsaturated $C_2$-$C_{20}$ alkyl group, optionally interrupted by one or more heteroatoms chosen from N, S and O, and/or optionally substituted by one or more identical or different groups chosen from:

i) -OR3,
ii) -SR3,
iii) -NR3R4,
iv) -CONHR3, or
v) -COOR3;

c) a saturated $C_1$-$C_8$ alkyl group substituted by at least one $C_5$-$C_{12}$ aryl radical, said aryl radical optionally being substituted by one or more hydroxyls and/or by one or more $C_1$-$C_8$ alkoxy radicals; or
d) a phenyl group optionally substituted by one or more hydroxyls and/or by one or more $C_1$-$C_8$ alkoxy radicals;

R3 denoting a hydrogen atom or a saturated linear $C_1$-$C_6$ or branched $C_3$-$C_6$ or unsaturated $C_2$-$C_5$ alkyl group,
R4 denoting a hydrogen atom, a saturated linear $C_1$-$C_5$ or branched $C_3$-$C_5$ hydrocarbon group, or an acetyl group;
it being possible for R1 and R2 to form, with the nitrogen atom which bears them, a ring chosen from pyrrolidine, pyrroline, piperidine, piperazine, morpholine, thiomorpholine and azepine;
and also their salts, their solvates, their optical isomers and their racemates,
as inhibitor of the black or brown colouration of comedones.

2. Use according to Claim 1, the compound corresponding to the formula (I) or (1'), in which formulae:

R1 denotes a radical chosen from:

a) a hydrogen atom, or
b) a saturated linear $C_1$-$C_{10}$ or branched $C_3$-$C_{10}$ or unsaturated $C_2$-$C_{10}$ alkyl group, optionally substituted by one or more -OR3 groups; and

R2 denotes a radical chosen from:

a) a hydrogen atom,
b) a saturated linear $C_1$-$C_{10}$ or branched $C_3$-$C_{10}$ or cyclic $C_3$-$C_7$ alkyl group, optionally interrupted by one or more oxygen atoms and/or optionally substituted by one or more identical or different groups chosen from:

i) -OR3,
ii) -NR3R4,
iii) -CONHR3, or
iv) -COOR3,

c) a saturated $C_1$-$C_6$ alkyl group substituted by a phenyl radical optionally substituted by one or more hydroxyls and/or by one or more $C_1$-$C_3$ alkoxy radicals,
d) a phenyl group optionally substituted by one or more hydroxyls and/or by one or more $C_1$-$C_3$ alkoxy radicals,

R3 denoting a hydrogen atom or a saturated linear $C_1$-$C_5$ or branched $C_3$-$C_5$ hydrocarbon group;
R4 denoting a hydrogen atom or a saturated linear $C_1$-$C_5$ or branched $C_3$-$C_5$ hydrocarbon group;
and also their salts, their solvates, their optical isomers and their racemates.

**3.** Use according to Claim 1 or 2, the compound corresponding to the formula (I) or (I'), in which formulae:

R1 denotes a hydrogen atom or a $C_1$-$C_6$ hydroxyalkyl group; and
R2 denotes:

a) a hydrogen atom,
b) a saturated linear $C_1$-$C_{10}$ or branched $C_3$-$C_{10}$ alkyl group, or
c) a saturated $C_1$-$C_6$ alkyl group substituted by a phenyl radical, in particular a benzyl group, and also their salts, their solvates, their optical isomers and their racemates.

**4.** Use according to Claim 1 or 2, the compound corresponding to the following formula (II) or (II'):

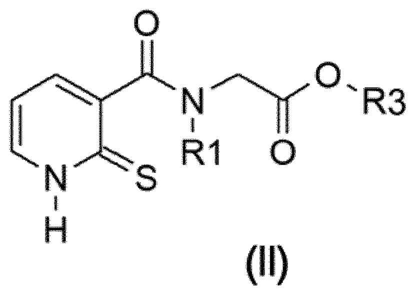
(II)

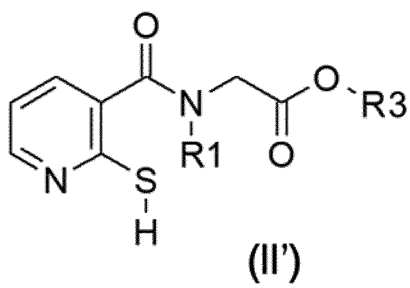
(II')

in which:

R1 denotes a radical chosen from:

a) a hydrogen atom, or
b) a saturated linear $C_1$-$C_4$ alkyl radical, preferably a methyl radical; and

R3 denotes a radical chosen from:

a) a hydrogen atom, or
b) a saturated linear $C_1$-$C_4$ alkyl group, preferably an ethyl group, or branched $C_3$-$C_4$ alkyl group, preferably an isopropyl and isobutyl group,

and also their salts, their solvates, their optical isomers and their racemates.

**5.** Use according to any one of the preceding claims, in which said compound of formula (I) or (I’) is incorporated in a cosmetic composition intended for a topical application.

**6.** Use according to Claim 5, in which said compound of formula (I) or (I’) is present in the cosmetic composition in a content ranging from 0.1% to 5.0% by weight, preferably from 0.1% to 3.0% by weight, in particular from 0.5% to 3.0% by weight, with respect to the total weight of the composition.

**7.** Use according to either of Claims 5 and 6, in which the cosmetic composition additionally comprises at least one additional active principle, in particular chosen from salicylic acid, pro-xylane, retinol and their mixtures.

**8.** Cosmetic method for preventing the black or brown colouration of the comedones, comprising the topical application, to the skin, of a cosmetic composition comprising at least one compound corresponding to the formula (I) or (I’), which formulae are as defined in any one of Claims 1 to 4.

**9.** Cosmetic method according to Claim 8, in which the skin is the skin of the face and/or of the body, in particular of the face and/or of the hands, preferably of the face, and more particularly of the forehead and/or of the alae of the nose and/or of the chin.

**10.** Cosmetic method according to Claim 8 or 9 for preventing the black or brown colouration of the comedones, comprising:

a) a stage of topical application, to the skin, of a cosmetic composition comprising at least one compound corresponding to the formula (I) or (I’), which compound is as defined in any one of Claims 1 to 4, and
b) a stage of topical application of a composition different from the composition applied in stage a) and containing at least one active principle distinct from the compounds corresponding to the formula (I) or (1’).

**Patentansprüche**

**1.** Kosmetische Verwendung von mindestens einer Verbindung der folgenden Formel (I) oder (I’):

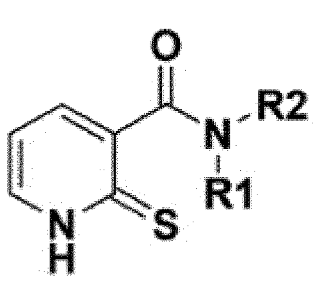
(I)

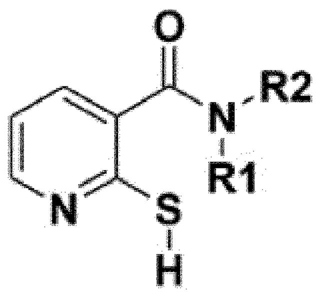
(I’)

in welchen Formeln:

R1 und R2, die gleich oder verschieden sind, einen Rest bezeichnen, der ausgewählt ist aus:

a) einem Wasserstoffatom;
b) einer gesättigten linearen $C_1$-$C_{20}$- oder verzweigten $C_3$-$C_{20}$- oder ungesättigten $C_2$-$C_{20}$-Alkylgruppe,

optional unterbrochen durch ein oder mehrere Heteroatome, ausgewählt aus N, S und O,
und/oder optional substituiert durch eine oder mehrere gleiche oder verschiedene Gruppen, ausgewählt aus:

i) -OR3,
ii) -SR3,
iii) -NR3R4,
iv) -CONHR3, oder
v) -COOR3;

c) einer gesättigten $C_1$-$C_8$-Alkylgruppe, die durch mindestens einen $C_5$-$C_{12}$-Arylrest substituiert ist, wobei der Arylrest optional durch einen oder mehrere Hydroxylreste und/oder durch einen oder mehrere $C_1$-$C_8$-Alkoxyreste substituiert ist; oder
d) einer Phenylgruppe, die optional durch eine oder mehrere Hydroxylreste und/oder durch einen oder mehrere $C_1$-$C_8$-Alkoxyreste substituiert ist;
R3 ein Wasserstoffatom oder eine gesättigte lineare $C_1$-$C_6$- oder verzweigte $C_3$-$C_6$- oder ungesättigte $C_2$-$C_5$-Alkylgruppe bezeichnet,
R4 ein Wasserstoffatom, eine gesättigte linearen $C_1$-$C_5$- oder verzweigte $C_3$-$C_5$-Kohlenwasserstoffgruppe oder eine Acetylgruppe bezeichnet;
wobei R1 und R2 mit dem Stickstoffatom, das sie trägt, einen Ring bilden können, der aus Pyrrolidin, Pyrrolin, Piperidin, Piperazin, Morpholin, Thiomorpholin und Azepin ausgewählt ist;
sowie ihre Salze, ihre Solvate, ihre optischen Isomere und ihre Racemate,
als Inhibitor für die Schwarz- oder Braunfärbung von Komedonen.

**2.** Verwendung nach Anspruch 1, wobei die Verbindung der Formel (I) oder (I') entspricht, in welchen Formeln:

R1 einen Rest bezeichnet, ausgewählt aus:

a) einem Wasserstoffatom, oder
b) einer gesättigten linearen $C_1$-$C_{10}$- oder verzweigten $C_3$-$C_{10}$- oder ungesättigten $C_2$-$C_{10}$-Alkylgruppe, die optional durch eine oder mehrere -OR3-Gruppen substituiert ist; und

R2 einen Rest bezeichnet, ausgewählt aus:

a) einem Wasserstoffatom,
b) einer gesättigten linearen $C_1$-$C_{10}$- oder verzweigten $C_3$-$C_{10}$- oder zyklischen $C_3$-$C_7$-Alkylgruppe, die optional durch ein oder mehrere Sauerstoffatome unterbrochen und/oder optional durch eine oder mehrere gleiche oder verschiedene Gruppen substituiert ist, ausgewählt aus:

i) -OR3,
ii) -NR3R4,
iii) -CONHR3, oder
iv) -COOR3,

c) einer gesättigten $C_1$-$C_6$-Alkylgruppe, die durch einen Phenylrest substituiert ist, der optional durch einen oder mehrere Hydroxylreste und/oder durch einen oder mehrere $C_1$-$C_3$-Alkoxyreste substituiert ist,
d) einer Phenylgruppe, die optional durch eine oder mehrere Hydroxylreste und/oder einen oder mehrere $C_1$-$C_3$-Alkoxyreste substituiert ist,

R3 ein Wasserstoffatom oder eine gesättigte lineare $C_1$-$C_5$- oder verzweigte $C_3$-$C_5$-Kohlenwasserstoffgruppe bezeichnet;
R4 ein Wasserstoffatom oder eine gesättigte lineare $C_1$-$C_5$- oder eine verzweigte $C_3$-$C_5$-Kohlenwasserstoffgruppe bezeichnet;
sowie ihre Salze, ihre Solvate, ihre optischen Isomere und ihre Racemate.

**3.** Verwendung nach Anspruch 1 oder 2, wobei die Verbindung der Formel (I) oder (I') entspricht, in welchen Formeln:

R1 ein Wasserstoffatom oder eine $C_1$-$C_6$-Hydroxyalkylgruppe bezeichnet; und
R2 bezeichnet:

a) ein Wasserstoffatom,
b) eine gesättigte lineare $C_1$-$C_{10}$- oder verzweigte $C_3$-$C_{10}$-Alkylgruppe, oder

c) eine gesättigte $C_1$-$C_6$-Alkylgruppe, die durch einen Phenylrest substituiert ist, insbesondere eine Benzylgruppe,

sowie ihre Salze, ihre Solvate, ihre optischen Isomere und ihre Racemate.

**4.** Verwendung nach Anspruch 1 oder 2, wobei die Verbindung der folgenden Formel (II) oder (II') entspricht:

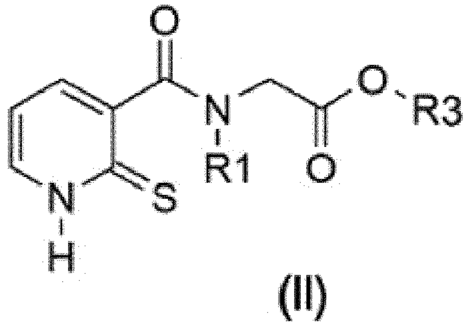

(II)

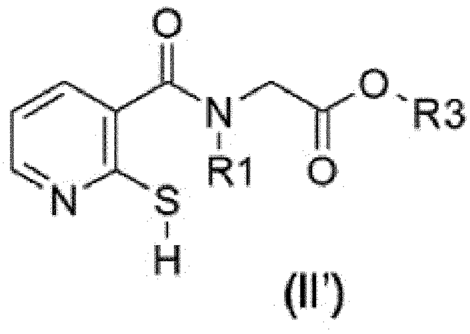

(II')

in denen:

R1 einen Rest bezeichnet, ausgewählt aus:

a) einem Wasserstoffatom, oder
b) einem gesättigten linearen $C_1$-$C_4$-Alkylrest, vorzugsweise einem Methylrest; und

R3 einen Rest bezeichnet, ausgewählt aus:

a) einem Wasserstoffatom, oder
b) einer gesättigten linearen $C_1$-$C_4$-Alkylgruppe, vorzugsweise einer Ethylgruppe, oder einer verzweigten $C_3$-$C_4$-Alkylgruppe, vorzugsweise einer Isopropyl- und Isobutylgruppe,

sowie ihre Salze, ihre Solvate, ihre optischen Isomere und ihre Racemate.

**5.** Verwendung nach einem der vorhergehenden Ansprüche, **dadurch gekennzeichnet, dass** die Verbindung der Formel (I) oder (I') in einer kosmetischen Zusammensetzung enthalten ist, die für eine topische Anwendung bestimmt ist.

**6.** Verwendung nach Anspruch 5, **dadurch gekennzeichnet, dass** die Verbindung der Formel (I) oder (I') in der kosmetischen Zusammensetzung in einem Gehalt von 0,1 bis 5,0 Gew.-% enthalten ist, vorzugsweise von 0,1 bis 3,0 Gew.-%, insbesondere von 0,5 bis 3,0 Gew.-%, bezogen auf das Gesamtgewicht der Zusammensetzung.

**7.** Verwendung nach einem der Ansprüche 5 und 6, **dadurch gekennzeichnet, dass** die kosmetische Zusammensetzung zusätzlich mindestens einen weiteren Wirkstoff umfasst, insbesondere ausgewählt aus Salicylsäure, Pro-Xylan, Retinol und deren Mischungen.

**8.** Kosmetisches Verfahren zur Verhinderung der Schwarz- oder Braunfärbung von Komedonen, umfassend die topische Anwendung einer kosmetischen Zusammensetzung auf die Haut, die mindestens eine Verbindung entsprechend der Formel (I) oder (I') umfasst, wobei die Formeln wie in einem der Ansprüche 1 bis 4 definiert sind.

**9.** Kosmetisches Verfahren nach Anspruch 8, bei dem die Haut die Haut des Gesichts und/oder des Körpers, insbesondere des Gesichts und/oder der Hände, vorzugsweise des Gesichts, und insbesondere der Stirn und/oder der Nasenflügel und/oder des Kinns ist.

**10.** Kosmetisches Verfahren nach Anspruch 8 oder 9 zur Verhinderung der Schwarz- oder Braunfärbung von Komedonen, umfassend:

a) eine Stufe einer topischen Anwendung einer kosmetischen Zusammensetzung auf die Haut, die mindestens

eine Verbindung entsprechend der Formel (I) oder (I') umfasst, wobei die Verbindung wie in einem der Ansprüche 1 bis 4 definiert ist, und

b) eine Stufe einer topischen Anwendung einer Zusammensetzung, die sich von der in Stufe a) angewandten Zusammensetzung unterscheidet und mindestens einen von den Verbindungen der Formel (I) oder (I') verschiedenen Wirkstoff enthält.

**Revendications**

1. Utilisation cosmétique d'au moins un composé correspondant à l'une des formules (I) ou (I') suivantes :

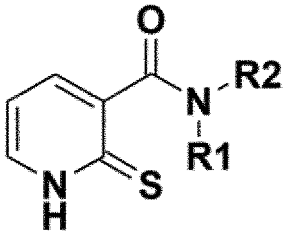

(I)

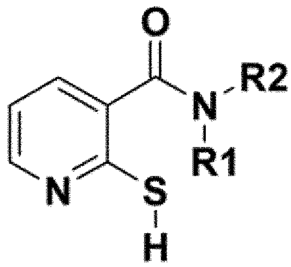

(I')

formules dans lesquelles :

R1 et R2, qui sont identiques ou différents, désignent un radical choisi parmi :

a) un atome d'hydrogène ;

b) un groupe alkyle saturé linéaire en $C_1$ à $C_{20}$ ou ramifié en $C_3$ à $C_{20}$ ou insaturé en $C_2$ à $C_{20}$, éventuellement interrompu par un ou plusieurs hétéroatomes choisis parmi N, S et O, et/ou éventuellement substitué par un ou plusieurs groupes identiques ou différents choisis parmi :

i) -OR3,
ii) -SR3,
iii) -NR3R4,
iv) -CONHR3, ou
v) -COOR3 ;

c) un groupe alkyle saturé en $C_1$ à $C_8$ substitué par au moins un radical aryle en $C_5$ à $C_{12}$, ledit radical aryle étant éventuellement substitué par un ou plusieurs hydroxyles et/ou par un ou plusieurs radicaux alcoxy en $C_1$ à $C_8$ ; ou

d) un groupe phényle éventuellement substitué par un ou plusieurs hydroxyles et/ou par un ou plusieurs radicaux alcoxy en $C_1$ à $C_8$ ;

R3 désignant un atome d'hydrogène ou un groupe alkyle saturé linéaire en $C_1$ à $C_6$ ou ramifié en $C_3$ à $C_6$ ou insaturé en $C_2$ à $C_5$,

R4 désignant un atome d'hydrogène, un groupe hydrocarboné saturé linéaire en $C_1$ à $C_5$ ou ramifié en $C_3$ à $C_5$, ou un groupe acétyle ;

étant possible que R1 et R2 forment, avec l'atome d'azote qui les porte, un cycle choisi parmi pyrrolidine, pyrroline, pipéridine, pipérazine, morpholine, thiomorpholine et azépine ;

et aussi leurs sels, leurs solvates, leurs isomères optiques et leurs racémates,

en tant qu'inhibiteur de la coloration noire ou marron des comédons.

2. Utilisation selon la revendication 1, le composé correspondant à l'une des formules (I) ou (I'), formules dans lesquelles :

R1 désigne un radical choisi parmi :

a) un atome d'hydrogène, ou

b) un groupe alkyle saturé linéaire en $C_1$ à $C_{10}$ ou ramifié en $C_3$ à $C_{10}$ ou insaturé en $C_2$ à $C_{10}$, éventuellement substitué par un ou plusieurs groupes -OR3 ; et

R2 désigne un radical choisi parmi :

a) un atome d'hydrogène,
b) un groupe alkyle linéaire en $C_1$ à $C_{10}$ ou ramifié en $C_3$ à $C_{10}$ ou cyclique en $C_3$ à $C_7$, éventuellement interrompu par un ou plusieurs atomes d'oxygène et/ou éventuellement substitué par un ou plusieurs groupes identiques ou différents choisis parmi :

i) -OR3,
ii) -NR3R4,
iii) -CONHR3, ou
iv) -COOR3,

c) un groupe alkyle en $C_1$ à $C_6$ saturé substitué par un radical phényle éventuellement substitué par un ou plusieurs hydroxyles et/ou par un ou plusieurs radicaux alcoxy en $C_1$ à C3,
d) un groupe phényle éventuellement substitué par un ou plusieurs hydroxyles et/ou par un ou plusieurs radicaux alcoxy en $C_1$ à $C_3$,

R3 désignant un atome d'hydrogène ou un groupe hydrocarboné saturé linéaire en $C_1$ à $C_5$ ou ramifié en $C_3$ à $C_5$ ;
R4 désignant un atome d'hydrogène ou un groupe hydrocarboné saturé linéaire en $C_1$ à $C_5$ ou ramifié en $C_3$ à $C_5$ ;
et aussi leurs sels, leurs solvates, leurs isomères optiques et leurs racémates.

**3.** Utilisation selon la revendication 1 ou 2, le composé correspondant à l'une des formules (I) ou (I'), formules dans lesquelles :

R1 désigne un atome d'hydrogène ou un groupe hydroxyalkyle en $C_1$ à $C_6$ ; et
R2 désigne :

a) un atome d'hydrogène,
b) un groupe alkyle saturé linéaire en $C_1$ à $C_{10}$ ou ramifié en $C_3$ à $C_{10}$, ou
c) un groupe alkyle en $C_1$ à $C_6$ saturé substitué par un radical phényle, en particulier un groupe benzyle,

et aussi leurs sels, leurs solvates, leurs isomères optiques et leurs racémates.

**4.** Utilisation selon la revendication 1 ou 2, le composé correspondant à l'une des formules (II) ou (II') :

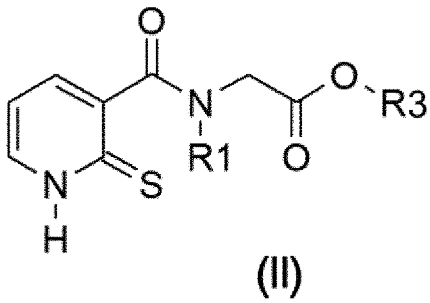

(II)

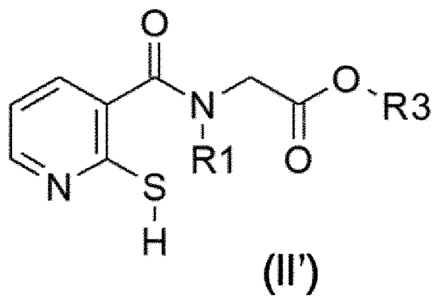

(II')

dans lesquelles :

R1 désigne un radical choisi parmi :

a) un atome d'hydrogène, ou
b) un radical alkyle saturé linéaire en $C_1$ à $C_4$, de préférence un radical méthyle ; et

R3 désigne un radical choisi parmi :

a) un atome d'hydrogène, ou
b) un groupe alkyle saturé linéaire en $C_1$ à $C_4$, de préférence un groupe éthyle, ou un groupe alkyle ramifié en $C_3$ à $C_4$, de préférence un groupe isopropyle et un groupe isobutyle,

et aussi leurs sels, leurs solvates, leurs isomères optiques et leurs racémates.

**5.** Utilisation selon l'une quelconque des revendications précédentes, dans laquelle ledit composé de formule (I) ou (I') est incorporé dans une composition cosmétique destinée à être appliquée par voie topique.

**6.** Utilisation selon la revendication 5, dans laquelle ledit composé de formule (I) ou (I') est présent dans la composition cosmétique à une teneur située dans la plage allant de 0,1 % à 5,0 % en poids, de préférence de 0,1 % à 3,0 % en poids, en particulier de 0,5 % à 3,0 % en poids, par rapport au poids total de la composition.

**7.** Utilisation selon l'une ou l'autre des revendications 5 et 6, dans laquelle la composition cosmétique comprend de plus au moins un principe actif additionnel, en particulier choisi parmi l'acide salicylique, le pro-xylane, le rétinol et leurs mélanges.

**8.** Procédé cosmétique pour empêcher la coloration noire ou marron des comédons, comprenant l'application topique à la peau d'une composition cosmétique comprenant au moins un composé correspondant à l'une des formules (I) ou (I'), lesquelles formules sont telles que définies dans l'une quelconque des revendications 1 à 4.

**9.** Procédé cosmétique selon la revendication 8, dans lequel la peau est la peau du visage et/ou du corps, en particulier du visage et/ou des mains, de préférence du visage, et plus particulièrement du front et/ou des ailes du nez et/ou du menton.

**10.** Procédé cosmétique selon la revendication 8 ou 9 pour empêcher la coloration noire ou marron des comédons, comprenant :

a) une étape d'application topique à la peau d'une composition cosmétique comprenant au moins un composé correspondant à l'une des formules (I) ou (I'), lequel composé est tel que défini dans l'une quelconque des revendications 1 à 4, et
b) une étape d'application topique à la peau d'une composition différente de la composition appliquée dans l'étape a) et contenant au moins un principe actif distinct des composés correspondant à l'une des formules (I) ou (I').

[Fig 1]

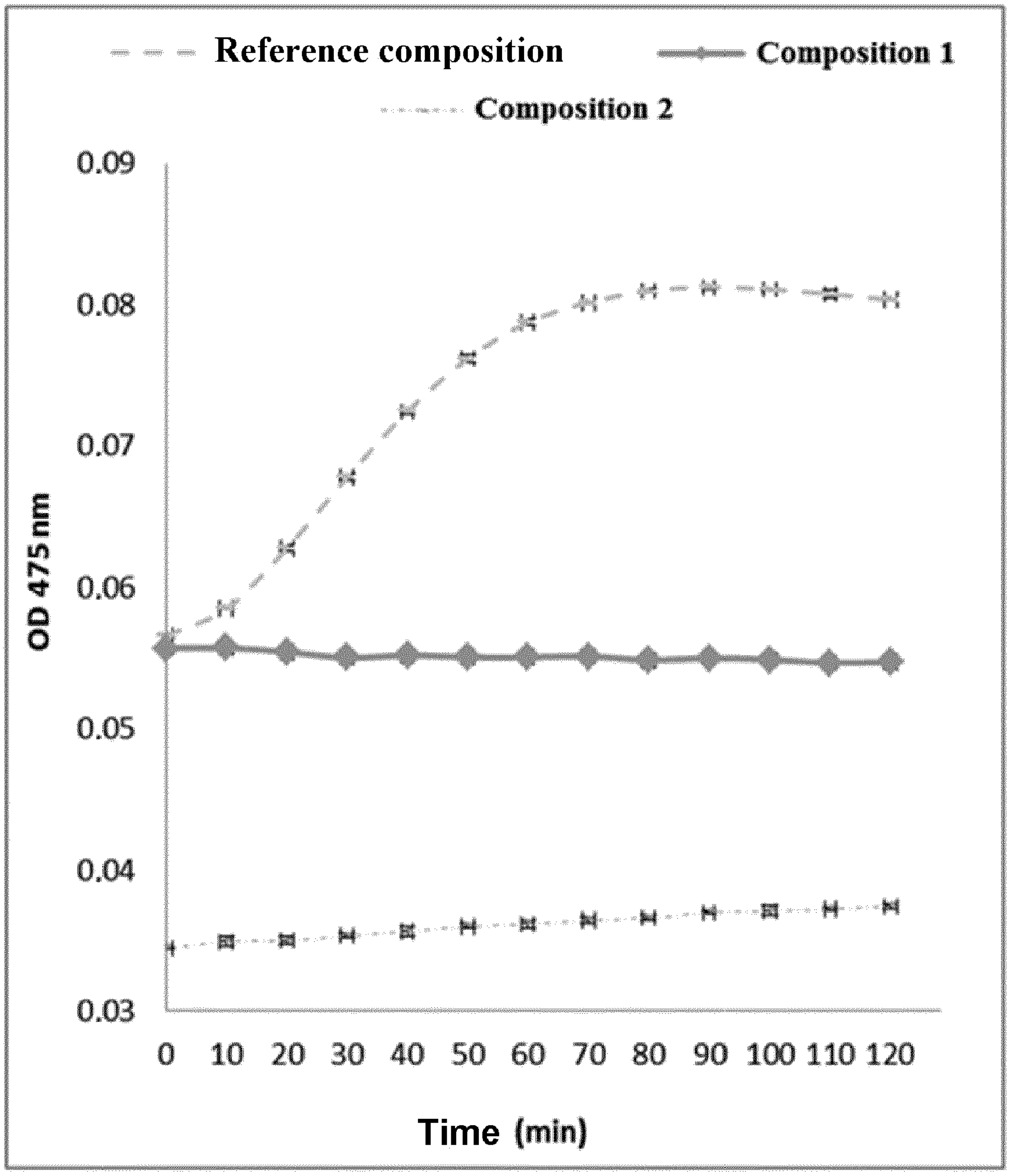
Reference composition
Composition 1
Composition 2
0.09
0.08
0.07
0.06
0.05
0.04
0.03
OD 475 nm
0
10
20
30
40
50
60
70
80
90
100
110
120
Time (min)

[Fig 2]

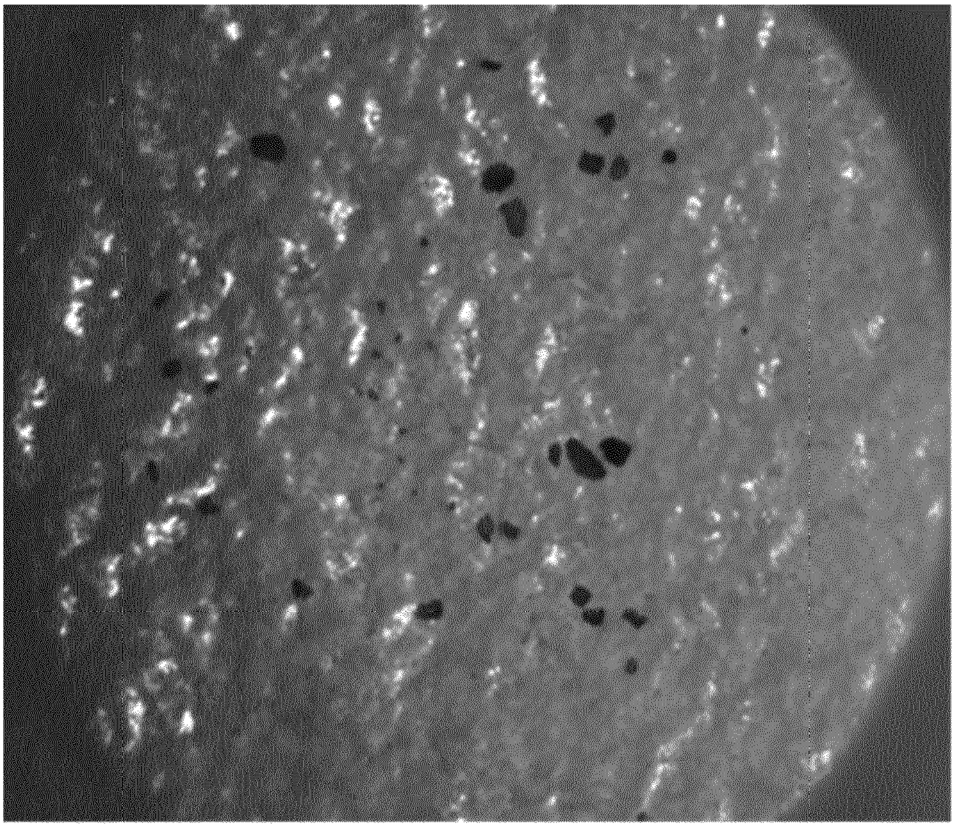

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FR 3044905 B1
- FR 2968661 B1
- FR 3045604 B1
- EP 298752 A
- WO 03014062 A
- FR 2349591 A
- EP 2555450 A
- WO 2008012532 A
- WO 2012080075 A1
- WO 2017102349 A1


### Non-patent literature cited in the description


- Interconversion of Nitriles, Carboxylic Acids and Derivatives. **R. LAROCK.** Comprehensive Organic Transformations. Wiley VCH
- Phosphorus in organic synthesis - XI, Amino acids and peptides -XXI, Reaction of diethyl phosphorocyanidate with carboxylic acids. A new synthesis of carboxylic esters and amides. *Tetrahedron,* 1976, vol. 32, 2211-2217
- **A. MONGE ; V. MARTINEZ-MERINO;.** Synthesis of 2-substituted 3-Oxoisothiazolo[5,4-b]pyridines;. *J. Heterocyclic. Chem.,* 1985, vol. 22, 1353
- **A. DUNN ; R. NORRIE;.** Synthesis of pyrido-1,3-thiazines;. *Zeitschrift fur Chemie,* 1988, vol. 28 (6), 212, , 214
- **S. ANDREAE;.** *J. Prakt. Chem.,* 1997, vol. 339, 152-158
- **S. GORSUCH;.** *Biorganic & Medicinal Chemistry,* 2009, vol. 17, 467-474
- **A. MONGE et al.** *J. Heterocyclic Chem.,* 1988, vol. 25, 23
- **M. PREGNOLATO;.** *Il Farmaco,* 2000, vol. 55, 669-679
- **BEHBAHANI et al.** *Microchemical Journal,* 1993, vol. 47, 251